Europäisches Patentamt

European Patent Office

Office européen des brevets

(11) Publication number: **0 294 940 B1**

# EUROPEAN PATENT SPECIFICATION

(45) Date of publication of patent specification:
**11.12.91 Bulletin 91/50**

(51) Int. Cl.⁵: **A61K 31/47,** // (A61K31/47,
31:46)

(21) Application number: **88304193.1**

(22) Date of filing: **09.05.88**

(54) Pharmaceutical compositions.

(30) Priority: **23.05.87 GB 8712251**

(43) Date of publication of application:
**14.12.88 Bulletin 88/50**

(45) Publication of the grant of the patent:
**11.12.91 Bulletin 91/50**

(84) Designated Contracting States:
**DE ES NL SE**

(56) References cited:
**GB-A- 2 022 078**
**GB-A- 2 157 291**

(73) Proprietor: **FISONS plc**
**Fison House Princes Street**
**Ipswich Suffolk IP1 1QH (GB)**

(72) Inventor: **Edwards, Alan Martin**
**Stable Cottage Pickwell**
**Melton Mowbray Leicestershire LE14 2RA**
**(GB)**
Inventor: **Rocchiccioli, Karen Mary Shannon**
**219 Park Road**
**Timperley Altrincham (GB)**

(74) Representative: **Wright, Robert Gordon McRae**
**FISONS plc 12 Derby Road**
**Loughborough Leicestershire LE11 0BB (GB)**

EP 0 294 940 B1

## Description

This invention relates to pharmaceutical mixtures and methods for their preparation.

British Patent Specification No GB-A-2022078 discloses a number of pyranoquinolines including 9-ethyl-6,9-dihydro-4,6-dioxo-10-propyl-4H-pyrano[3,2-g]quinoline-2,8-dicarboxylic acid, the disodium salt of which, nedocromil sodium, is useful in the treatment of, inter alia, reversible obstructive airways disease. The mode of action of nedocromil sodium is believed to involve the prevention or inhibition of the activation of mast cells by various stimuli including allergens.

Other drugs which are useful in the treatment of reversible obstructive airways disease are the so-called anticholinergic agents. The mechanism of action of such drugs is quite different to that of nedocromil sodium; they exhibit a bronchodilatory effect due to their antagonism of the binding of acetylcholine, liberated from vagal nerve endings, to the muscarinic receptors of bronchial smooth muscle.

We have now found that mixtures of nedocromil sodium with anticholinergic agents at the appropriate therapeutic ratio, possess the advantage that they are both palliative and prophylactic, are more effective, suffer less from loss of responsiveness, produce fewer side effects, reduce bronchial hyperactivity, can be used at lower doses, can be administered directly to the site of the disease, eg by inhalation, can be administered less frequently, eg 1-2 times per day, can be administered as a long term therapy, are longer acting, are more stable, are synergistic, cause better patient compliance, have a less offensive taste or possess other desirable properties as compared to the anticholinergic agent when used on its own, nedocromil sodium when used on its own or certain other mixtures when tested in relevant pharmacological models.

According to the invention there is provided a pharmaceutical mixture comprising

a) one or more of 9-ethyl-6,9-dihydro-4,6-dioxo-10-propyl-4H-pyrano[3,2-g]quinoline-2,8-dicarboxylic acid and pharmaceutically acceptable salts thereof ('active ingredient A') in combination with

b) one or more anticholinergic agents or pharmaceutically acceptable derivatives thereof ('active ingredient B').

We prefer to use the disodium salt of 9-ethyl-6,9-dihydro-10-propyl-4H-pyrano-[3,2-g]quinoline-2,8-dicarboxylic acid, which is known as nedocromil sodium. We also prefer to use only one active ingredient A in admixture with one anticholinergic agent as active ingredient B.

Specific anticholinergic agents that may be mentioned include atropine, pharmaceutically acceptable salts thereof, ipratropium bromide and oxitropium bromide.

According to the invention there is also provided a salt of 9-ethyl-6,9-dihydro-4,6-dioxo-10-propyl-4H-pyrano[3,2-g]quinoline-2,8-dicarboxylic acid with an anticholinergic agent which is basic or is, or is capable of forming, a cation.

The salt may be made by a metathetical process, eg by reacting a suitable salt, such as the disodium salt, of 9-ethyl-6,9-dihydro-4,6-dioxo-10-propyl-4H-pyrano[3,2-g] quinoline-2,8-dicarboxylic acid with an appropriate salt, eg the hydrochloride, sulphate or bromide of the anticholinergic agent. However, in the case of a basic anticholinergic agent, the salt is preferably produced by reacting the free acid, 9-ethyl-6,9-dihydro-4,6-dioxopropyl-4H-pyrano[3,2-g]quinoline-2,8-dicarboxylic acid with the free base of the anticholinergic agent, as such a process does not produce a by-product inorganic salt. The reaction may be carried out in a solvent which is inert under the reaction conditions. The solvent is preferably one in which the desired salt is soluble, eg water. The desired salt may be isolated and purified by, for example, crystallisation or by freeze drying.

According to the invention we therefore provide the salt when not in solution, eg the salt when in a substantially dry form, or when in admixture with insufficient liquid, eg water, to dissolve it all.

The salt may, if desired, be used in conjunction with one or more other salts of 9-ethyl-6,9-dihydro-4,6-dioxo-10-propyl-4H-pyrano[3,2-g]quinoline-2,8-dicarboxylic acid, notably the disodium salt thereof.

The ratio of active ingredients A and B in the composition can vary over a wide range, depending on, amongst other factors, the particular active ingredients used and the specific purpose for which the composition is intended. However we prefer the composition to contain from 0.4 to 400 parts by weight, and more preferably from 1 to 200 parts by weight of active ingredient A (measured as nedocromil sodium) for each part by weight of active ingredient B. When the active ingredients are present in the form of a salt of 9-ethyl-6,9-dihydro-10-propyl-4H-pyrano[3,2-g]quinoline-2,8-dicarboxylic acid with a basic or cationic anticholinergic agent, it may be necessary to use, in addition, no, or only a very small proportion of, 'free' anticholinergic agent.

A suitable dose of active ingredient A for inhalation is in the range 1 to 100mg and preferably 1 to 20 mg, (measured as nedocromil sodium).

It is much preferred that the dose of ingredient B be such as to give a sustained rather than a transitory action.

The mixture may be administered as divided doses from 1 to 6, and preferably 2 to 4, times per day. Each dose may comprise one or more unit doses.

2

The mixtures according to the invention may be made by mixing together the various active ingredients using known conventional techniques.

The salts and mixtures of the invention are preferably administered in admixture with a pharmaceutically acceptable carrier.

Thus, according to another aspect of the invention, there is provided a pharmaceutical composition comprising a salt or mixture according to the invention in admixture with a pharmaceutically acceptable carrier.

The composition preferably comprises less than 80%, and more preferably less than 50%, by weight of the salt or mixture.

The compositions of the invention may be administered by a wide variety of routes and may act systemically or locally. Thus, the compounds may be administered directly to the nose or eye, to the buccal cavity, oesophageally or to other available surfaces of the body. The new salts or mixtures may be administered directly to the organ or part of the body showing symptoms or to a part remote from that showing symptoms. We prefer administration to be by oral or nasal administration to the lung. Compositions suitable for administration by this route include solutions (especially aqueous solutions) for administration by nebulisation, and pressurised and non-pressurised powder compositions.

For use in powder compositions, the salts or mixtures are preferably in the form of particles having a mass median diameter of from about 0.01 to 10 µm, more preferably from 2 to 6 µm, and most preferably from 2 to 4 µm. Preferred anticholinergic agents for use in powder compositions include ipratropium bromide.

Non-pressurised powder compositions may contain an inert carrier, eg coarse lactose. Pressurised powder compositions may contain a pressurised gas, eg nitrogen, or a liquified propellant, the composition containing from about 1 to 20% w/w of the salt or mixture.

Examples of suitable carriers for compositions to be administered by other routes are :

for tablets, capsules and dragees — microcrystalline cellulose, calcium phosphate, diatomaceous earth, a sugar (eg lactose, dextrose or mannitol), talc, stearic acid, starch, sodium bicarbonate and/or gelatin ; and

for suppositories — natural or hardened oil or waxes.

The salts, mixtures and compositions of the invention are useful because they possess pharmacological activity in animals ; in particular they are useful because they inhibit the release and/or action of pharmacological mediators which result from the in vivo combination of certain types of antibody and specific antigen, eg the combination of reaginic antibody with specific antigen (see Example 27 of British Patent Specification No GB-A-1,292,601). The salts and compositions have also been found to inhibit the degranulation of mast cells and to interfere with reflex pathways in experimental animals and man, in particular those reflexes associated with lung function. In man, both subjective and objective changes which result from the inhalation of specific antigen by sensitised subjects are inhibited by prior administration of the new salts and compositions. Thus the new salts and compositions are useful in the treatment of reversible airway obstruction and/or to prevent the secretion of excess mucous. They are thus useful for the treatment of allergic asthma, so-called 'intrinsic' asthma (in which no sensitivity to extrinsic antigen can be demonstrated), exercise induced asthma, rhinitis, farmer's lung, bird fancier's disease, bronchitis, coughs (including whooping cough) and the nasal and bronchial obstructions associated with the common cold. The new salts, mixtures and compositions are also of value in the treatment of other conditions in which antigen-antibody reactions or excess mucous secretion are responsible for, or are an adjunct to, disease.

The pharmaceutical mixture according to the invention may be used in the treatment of reversible obstructive airways disease in which active ingredient A and active ingredient B are administered simultaneously or separated in time to a patient suffering from such a disease.

The method of treatment is preferably carried out on a regular daily basis, eg 1-4 times per day. The method is preferably carried out over a prolonged period, eg at least 4 weeks, more preferably at least 8 weeks. The method preferably comprises simultaneously administering fixed doses of active ingredient A with fixed doses of active ingredient B.

Active ingredients A and B may be supplied in a form containing both in combination or in a form containing the two active ingredients separately with indications or directions for their sequential administration in accordance with the method of treatment.

The new salts, mixtures and compositions of the invention may be used in a variety of dosing schedules, either on their own or in conjunction with one or more other active compounds.

The compositions or the invention are illustrated, by the following Examples 1 to 3. Typical experimental procedures in which activity of the salts, mixtures and compositions of the invention may be demonstrated are illustrated in Examples A to E.

3

## Example 1

### Nebuliser Solution

| | |
|---|---|
| Nedocromil sodium | 0.5% w/v |
| (Active ingredient A) | |
| Atropine methonitrate | 0.2% |
| (Active ingredient B) | |
| Purified water BP | to 100% |

Prepared by dissolving active ingredients A and B in the water.

## Example 2

### Pressurised Powder Formulation

| | |
|---|---|
| Nedocromil sodium | 0.5% w/w |
| (Active ingredient A) | |
| Ipratropium bromide | 0.2% |
| (Active ingredient B) | |
| Sorbitan trioleate | 0.5% |
| Propellant 114 | 39.2% |
| Propellant 12 | 58.8% |

The sorbitan ester is dispersed in up to half the propellant 12 at −40°C while stirring with a high dispersion mixer. The active ingredients are added to the resulting dispersion. The balance of the propellant 12 is then added at −50°C, followed by propellant 14 also cooled to −50°C. The resulting mixtures are then filled into vials onto which metering valves are subsequently crimped.

## Example 3

### Non-Pressurised Powder Formulation

| | |
|---|---|
| Nedocromil sodium | 40 % w/w |
| (Active ingredient A) | |
| Ipratropium bromide | 10% |
| (Active ingredient B) | |
| Lactose | 50% |

The active ingredients (particle size 0.01 to 10 μm) and the lactose (particle size 30 to 80 μm) are intimately mixed and then filled into hard gelatine capsules.

4

## Example A

### Rat Passive Lung Anaphylaxis Test

Female rats (Charles River) weighing 250 g were passively sensitised by injecting intravenously 0.5 ml of potentiated rat IgE antiserum raised against egg albumen.

The rats were anaesthetised with sodium pentobarbitone (70 mg/kg) intravenously. Tne trachea was cannulated and the rat ventilated with a Palmer Miniature Ideal respiratory pump in a closed system. Tracheal pressure was recorded through a side arm of the cannula connected to a differential air pressure transducer (UPI, Pye Ether Ltd). The respiratory rate was set at 99 strokes per minute with a tracheal pressure of 6 cm water.

The rats were challenged, 48 hours after sensitisation, with egg albumen (25 mg/kg, intravenously) and the subsequent increase in tracheal pressure expressed as a percentage of maximum possible degree of bronchoconstriction ($\Delta$ PCB max), measured by clamping the trachea.

Active ingredient A and/or active ingredient B either alone or in combination, were administered intravenously in saline over the period 1 to 5 minutes before antigen challenge.

The results of giving the two compounds in combination were analysed using an analysis of variance for a factorial design with repeated measures in each cell. A square root transformation was carried out prior to the analysis.

Any antagonistic or synergistic effect of the two compounds was further examined using the method described by Berenbaum, Clin Exp Immunol, 28, 1 (1977). This consists of calculating the value :

$$Z = \frac{[A]}{[Ae]} + \frac{[B]}{[Be]}$$

Where : [A] is the dose of A in the combination of A and B giving an effect X.
[B] is the dose of B in the combination of A and B giving an effect X.
[Ae] is the dose of A alone having an effect X.
[Be] is the dose of B alone having an effect X.

If : Z > 1, A and B are synergistic.
Z = 1, A and B are additive.
Z < 1, A and B are antagonistic.

## Example B

### Inhibition of mediator release from bronchoalveolar cells

Materials [³H]-S-Adenosyl-L-Methionine (500 mCi/mmol) and [³²P]-orthophosphate (carrier free) were obtained from A mersham International (Amersham, Buckinghamshire, UK), sheep antiserum to rat IgE, goat antiserum to human IgE and normal goat serum from Miles Laboratories (Slough, Buckinghamshire, UK). The goat anti-human IgE and control serum were precipitated with 40% saturated ammonium sulphate (Largman et al, Methods Enzymol (1981) 74 :22) and, after dialysis, the globul fraction adjusted to the original serum volume. The ascaris antigen was prepared from adult Ascaris suum pseudocoelomic fluid by chromatography on Sephadex G200 (Pharmacia, Milton Keynes, Buckinghamshire, UK), using the method of Ambler et al, J Immunol Meth (1972), 1, 317.

### Infection and bronchoalveolar lavage of macaques

A group of 20 Macaca arctoides monkeys, weight 8-18 kg, was used in these studies. The animals were infected with A. suum and lavaged as described in Pritchard et al, Clin Exp Immunol, (1983), 54 : 469. The lavage fluid was recovered into heparinised tubes and placed on ice.

### Treatment of BAL cells

The lavage fluid was filtered through a 175µm nylon mesh and the cells recovered by centrifugation (450 g for 5 minutes at 4°C). The cells were washed and resuspended to the required working dilution in buffer (10 mm HEPES-buffered Tyrode, pH 7.4 containing 1 mg/ml gelatin and 5 units/ml heparin). Differential cell counts were carried out in wet preparations in Kimura's stain (20). When only histamine release was to be measured, the cells were challenged in triplicate by adding 0.05 ml cells, at a density of $10^5$ mast cells/ml, to 0.05 ml buffer containing releasing agent and ingredient B (eg $3 \times 10^{-8} - 10^{-6}$M) and nedocromil sodium ($3 \times 10^{-6} - 10^{-4}$M) alone and in combination at 37°C. After incubating for 20 minutes at 37°C, the release process was quenched by the addition of 0.25 ml ice cold $Ca^{2+}$-, $Mg^{2+}$-free buffer containing 2 mM EDTA. After centrifugation (450 g for 5 minutes at 4°C), 0.2 ml supernatant was sampled for histamine analysis. In all inhibiting experiments a submaximal level of challenge was chosen which, from the experience of previous lavages of that animal, released 15-25% of the total histamine, but not more than half the maximum release attainable.

## Measurement of Histamine

Histamine was measured by the double isotope method of Beaven et al, Clin Chim Acta, (1972), <u>37</u>, 97 modified as described in Pritchard et al.

## Example C

## Non-specific Bronchial Hyperreactivity in Adult Asthmatics

Adult asthmatics gave informed consent and entered the study.

Following pretreatment with ingredients A and B alone or in combination, histamine challenge was performed by the method of Cockcroft, Clin Allergy, (1977), <u>7</u>, 235. Doubling concentrations of histamine acid phosphate (0.03-8 mg/ml) were inhaled for two minutes at five minute intervals. $FEV_1$ was measured on a Vitalograph dry wedge spirometer before and at 0.5 minutes and 1.5 minutes after each inhalation. The challenge was discontinued when $FEV_1$ had fallen by 20% or more from the baseline measure. Log-dose response curves were constructed and the $PC_{20}$ measured by interpolation of the last two points.

## Example D

Human volunteers suffering from specific allergic asthma were selected for the study. In these human volunteers an asthma attack normally follows the inhalation of an antigen to which he is specifically sensitive. The degree of asthma provoked by this method can be measured by repeated examination of the airway resistance.

A suitable designated spirometer is used to measure the forced expiratory volume at one second ($FEV_1$) hence the changes in airway resistance.

Drug induced changes in $FEV_1$ are measured at 5 minutes after administration of the drug.

Six hours after drug administration a standard antigen challenge is administered to the human volunteer and the fall in $FEV_1$ measured at 5 minutes after the antigen administration.

## Example E

## Effect of active ingredients A and B, separately and in combination in attenuating adenosine monophosphate induced bronchoconstriction

Atopic healthy volunteers were selected for the study. Prior to the administration of pretreatment, baseline values of forced expiratory volume in one second ($FEV_1$), partial expiratory flow rate (pEFR) and maximum expiratory flow rate (mEFR) were measured. Measurement was made on at least three occasions or until the readings were stable (less than 5% variation). On each occasion the pretreatment was inhaled for five minutes from a Wright Nebuliser (operating at a flow rate of 7 litres per minute) through the mouth during tidal breathing.

Prior to the commencement of adenosine monophosphate (AMP) challenge $FEV_1$, pEFR and mEFR were measured. The AMP challenge was performed on each study day by giving doubling doses of AMP, each inhaled for two minutes, from either a Wright Nebuliser (operating at a flow rate of 6.5 litres per minute) or an Acorn Nebuliser (operating at a flow rate of 5.5 litres per minute) until a reduction in pEFR of greater than 40% occurs. The nebuliser chosen for each subject was the same throughout the study. AMP was given in a 1 mg/ml-256 mg/ml concentration range, the diluent being physiological saline. Successive doses were administered at 5 minute intervals. Partial expiratory flow rates were measured at 0.5 and 2.5 minutes after each AMP dose. The dose giving a 40% ($PD_{40}$) reduction in pEFR was determined by interpolation from a log dose response curve.

The results given in Table 1 were obtained using the procedure of Example E in four volunteers. Ingredient A was nedocromil sodium administered as a 0.5% w/v aqueous solution ; ingredient B was atropine methonitrate administered as a 0.2% w/v aqueous solution. The combination was an aqueous solution containing 0.5% w/v nedocromil sodium and 0.2% w/v atropine methonitrate.

## Table 1

| Volunteer | $PD_{40}$ (mg/ml) | | |
|-----------|--------------|--------------|-------------|
|           | Ingredient A | Ingredient B | Combination |
| 1         | 43           | 42           | 128         |
| 2         | 32           | 23           | 128         |
| 3         | 17           | 12           | 44          |
| 4         | 27           | 3.4          | 119         |

## Claims

## Claims for the following Contracting States : DE, NL, SE

1. A synergetical pharmaceutical mixture comprising
a) one or more of 9-ethyl-6,9-dihydro-4,6-dioxo-10-propyl-4H-pyrano[3,2-g]quinoline-2,8-dicarboxylic acid and pharmaceutically acceptable salts thereof ("active ingredient A") in combination with
b) one or more anticholinergic agents or pharmaceutically acceptable derivatives thereof ("active ingredient B").
2. A pharmaceutical mixture according to Claim 1, wherein active ingredient A is nedocromil sodium.
3. A pharmaceutical mixture according to Claim 1 or Claim 2, wherein active ingredient B is atropine or a pharmaceutically acceptable derivative thereof.
4. A pharmaceutical mixture according to Claim 1 or Claim 2, wherein active ingredient B is ipratropium bromide.
5. A pharmaceutical mixture according to Claim 1 or Claim 2, wherein active ingredient B is oxitropium bromide.
6. A salt of 9-ethyl-6,9-dihydro-4,6-dioxo-10-propyl-4H-pyrano[3,2-g]quinoline-2,8-dicarboxylic acid with an anticholinergic agent which is basic or is, or is capable of forming, a cation.
7. A pharmaceutical composition comprising a mixture according to any one of Claims 1 to 5 or a salt according to Claim 6 in admixture with a pharmaceutically acceptable carrier.
8. A pharmaceutical composition according to Claim 7, which is an aqueous solution.
9. A pharmaceutical composition according to Claim 7, which is a pressurised or non-pressurised powder.
10. A pack containing active ingredients A and B, as defined in Claim 1, as a combined preparation for simultaneous or sequential use in the treatment of reversible obstructive airways disease.

## Claims for the following Contracting State : ES

1. A process for the preparation of a synergetical pharmaceutical mixture comprising
a) one or more of 9-ethyl-6,9-dihydro-4,6-dioxo-10-propyl-4H-pyrano[3,2-g]quinoline-2,8-dicarboxylic acid and pharmaceutically acceptable salts thereof ('active ingredient A') in combination with
b) one or more anticholinergic agents or pharmaceutically acceptable derivatives thereof ('active ingredient B'),
which process comprises mixing the ingredients.

7

2. A process according to Claim 1, wherein active ingredient A is nedocromil sodium.

3. A process for the preparation of a salt of 9-ethyl-6,9-dihydro-4,6-dioxo-10-propyl-4H-pyrano[3,2-g]quinoline-2,8-dicarboxylic acid with an anticholinergic agent which is basic or is, or is capable of forming, a cation, which process comprises reacting a suitable salt of 9-ethyl-6,9-dihydro-4,6-dioxo-10-propyl-4H-pyrano[3,2-g]quinoline-2,8-dicarboxylic acid with an appropriate salt of the anticholinergic agent.

4. A process according to Claim 3, wherein the anticholinergic agent is basic,
which process comprises reacting 9-ethyl-6,9-dihydro- 4,6-dioxo-propyl-4H-pyrano[3,2-g]quinoline-2,8-dicarboxylic acid with the free base of the anticholinergic agent.

5. A process for the preparation of a pharmaceutical composition comprising a mixture of

a) one or more of 9-ethyl-6,9-dihydro-4,6-dioxo-10-propyl-4H-pyrano[3,2-g]quinoline-2,8-dicarboxylic acid and pharmaceutically acceptable salts thereof ('active ingredient A') in combination with
b) one or more anticholinergic agents or pharmaceutically acceptable derivatives thereof ('active ingredient B'),
in admixture with a pharmaceutically acceptable carrier,
which process comprises bringing the active ingredients into admixture with the carrier.

6. A process for the preparation of a pharmaceutical composition comprising a salt of 9-ethyl-6,9-dihydro-4,6-dioxo-10-propyl-4H-pyrano[3,2-g]quinoline-2,8-dicarboxylic acid with an anticholinergic agent which is basic or is, or is capable of forming, a cation, in admixture with a pharmaceutically acceptable carrier,
which process comprises bringing the salt into admixture with the carrier.

7. A process according to Claim 6, wherein the composition is an aqueous solution.

8. A process according to Claim 5 or Claim 6, wherein the composition is a pressurised or non-pressurised powder.

## Patentansprüche

## Patentansprüche für folgende Vertragsstaaten : DE, NL, SE

1. Synergetische pharmazeutische Mischung, umfassend
a) ein oder mehrere von 9-Ethyl-6,9-dihydro-4,6-dioxo-10-propyl-4H-pyrano[3,2-g]chinolin-2,8-dicarbonsäure und pharmazeutisch annehmbare Salze hievon ("aktiver Inhaltsstoff A") in Kombination mit
b) einem oder mehreren anticholinergen Mitteln oder pharmazeutisch annehmbaren Derivaten hievon ("aktiver Inhaltsstoff B").

2. Pharmazeutische Mischung nach Anspruch 1, wobei der aktive Inhaltsstoff A Natriumnedocromil ist.

3. Pharmazeutische Mischung nach Anspruch 1 oder 2, wobei der aktive Inhaltsstoff B Atropin oder ein pharmazeutisch annehmbares Derivat hievon ist.

4. Pharmazeutische Mischung nach Anspruch 1 oder 2, wobei der aktive Inhaltsstoff B Ipratropiumbromid ist.

5. Pharmazeutische Mischung nach Anspruch 1 oder 2, wobei der aktive Inhaltsstoff B Oxitropiumbromid ist.

6. Salz der 9-Ethyl-6,9-dihydro-4,6-dioxo-10-propyl-4H-pyrano[3,2-g]chinolin-2,8-dicarbonsäure mit einem anticholinergen Mittel, das basisch oder dazu fähig ist, ein Kation zu bilden, oder ein Kation ist.

7. Pharmazeutische Zusammensetzung, umfassend eine Mischung nach einem der Ansprüche 1 bis 5 oder ein Salz nach Anspruch 6 in Mischung mit einem pharmazeutisch annehmbaren Träger.

8. Pharmazeutische Zusammensetzung nach Anspruch 7 in Form einer wässerigen Lösung.

9. Pharmazeutische Zusammensetzung nach Anspruch 7 in Form eines druckbeaufschlagten oder nicht-druckbeaufschlagten Pulvers.

10. Verpackung, enthaltend die aktiven Inhaltsstoffe A und B, wie in Anspruch 1 definiert, als kombinierte Zubereitung zur gleichzeitigen oder aufeinanderfolgenden Verwendung in der Behandlung reversibel obstruktiver Luftwegserkrankungen.

## Patentansprüche für folgende Vertragsstaaten : ES

1. Verfahren zur Herstellung einer synergetischen pharmazeutischen Mischung, umfassend
a) ein oder mehrere von 9-Ethyl-6,9-dihydro-4,6-dioxo-10-propyl4H-pyrano[3,2-g]chinolin-2,8-dicarbonsäure und pharmazeutisch annehmbare Salze hievon ("aktiver Inhaltsstoff A") in Kombination mit
b) einem oder mehreren anticholinergen Mitteln oder pharmazeutisch annehmbaren Derivaten hievon ("aktiver Inhaltsstoff B"), welches Verfahren das Mischen der Inhaltsstoffe umfaßt.

2. Verfahren nach Anspruch 1, wobei der aktive Inhaltsstoff A Natriumnedocromil ist.

3. Verfahren zur Herstellung eines Salzes der 9-Ethyl-6,9-dihydro-4,6-dioxo-10-propyl-4H-pyrano[3,2-g]chinolin-2,8-dicarbonsäure mit einem anticholinergen Mittel, das basisch oder dazu fähig ist, ein Kation zu bilden, oder ein Kation ist, welches Verfahren das Umsetzen eines geeigneten Salzes der 9-Ethyl-6,9-dihydro-4,6-dioxo-10-propyl-4H-pyrano[3,2-g]chinolin-2,8-dicarbonsäure mit einem entsprechenden Salz des anticholinergen Mittels umfaßt.

4. Verfahren nach Anspruch 3, wobei das anticholinerge Mittel basisch ist, welches Verfahren das Umsetzen der 9-Ethyl-6,9dihydro-4,6-dioxo-10-propyl-4H-pyrano[3,2-g]chinolin-2,8-dicarbonsäure mit der freien Base des anticholinergen Mittels umfaßt.

5. Verfahren zur Herstellung einer pharmazeutischen Zusammensetzung, umfassend eine Mischung von
a) einem oder mehreren von 9-Ethyl-6,9-dihydro-4,6-dioxo-10-propyl-4H-pyrano[3,2-g]chinolin-2,8-dicarbonsäure und pharmazeutisch annehmbare Salze hievon ("aktiver Inhaltsstoff A") in Kombination mit
b) einem oder mehreren anticholinergen Mitteln oder pharmazeutisch annehmbaren Derivaten hievon ("aktiver Inhaltsstoff B"), in Mischung mit einem pharmazeutisch annehmbaren Träger, welches Verfahren das Mischen der Inhaltsstoffe mit dem Träger umfaßt.

6. Verfahren zur Herstellung einer pharmazeutischen Zusammensetzung, umfassend ein Salz der 9-Ethyl-6,9-dihydro-4,6-dioxo-10-propyl-4H-pyrano[3,2-g]chinolin-2,8-dicarbonsäure mit einem anticholinergen Mittel, das basisch oder dazu fähig ist, ein Kation zu bilden, in Mischung mit einem pharmazeutisch annehmbaren Träger, welches Verfahren das Mischen des Salzes mit dem Träger umfaßt.

7. Verfahren nach Anspruch 6, wobei die Zusammensetzung eine wässerige Lösung ist.

8. Verfahren nach Anspruch 5 oder 6, wobei die Zusammensetzung ein druckbeaufschlagtes oder nicht-druckbeaufschlagtes Pulver ist.

## Revendications

### Revendications pour les Etats Contractants : DE, NL, SE

1. Mélange pharmaceutique synergique comprenant :
a) un ou plusieurs d'entre l'acide 9-éthyl-6,9-dihydro-4,6-dioxo-10-propyl-4H-pyrano[3,2-g]quinoléine-2,8-dicarboxylique et ses sels pharmaceutiquement acceptables ("constituant actif A") en combinaison avec
b) un ou plusieurs agents anticholinergiques ou leurs dérivés pharmaceutiquement acceptables ("constituant actif B").

2. Mélange pharmaceutique suivant la revendication 1, dans lequel le constituant actif A est le nédocromil sodique.

3. Mélange pharmaceutique suivant la revendication 1 ou 2, dans lequel le constituant actif B est l'atropine ou un dérivé pharmaceutiquement acceptable de celle-ci.

4. Mélange pharmaceutique suivant la revendication 1 ou 2, dans lequel le constituant actif B est le bromure d'ipratropium.

5. Mélange pharmaceutique suivant la revendication 1 ou 2, dans lequel le constituant actif B est le bromure d'oxitropium.

6. Sel de l'acide 9-éthyl-6,9-dihydro-4,6-dioxo10-propyl-4H-pyrano[3,2-g]quinoléine-2,8-dicarboxylique avec un agent anticholinergique qui est basique ou qui est un cation ou est capable d'en former un.

7. Composition pharmaceutique comprenant un mélange suivant l'une quelconque des revendications 1 à 5 ou un sel suivant la revendication 6 en mélange avec un excipient pharmaceutiquement acceptable.

8. Composition pharmaceutique suivant la revendication 7, qui est une solution aqueuse.

9. Composition pharmaceutique suivant la revendication 7, qui est une poudre mise ou non sous pression.

10. Emballage contenant les constituants actifs A et B tels que définis dans la revendication 1, sous la forme d'une préparation combinée pour l'utilisation simultanée ou successive dans le traitement de l'affection obstructive réversible des voies respiratoires.

### Revendications pour l'Etat Contractant : ES

1. Procédé de préparation d'un mélange pharmaceutique synergique comprenant :
a) un ou plusieurs d'entre l'acide 9-éthyl-6,9-dihydro-4,6-dioxo-10-propyl-4H-pyrano[3,2-g]quinoléine-2,8-dicarboxylique et ses sels pharmaceutiquement acceptables ("constituant actif A") en combinaison avec
b) un ou plusieurs agents anticholinergiques ou leurs dérivés pharmaceutiquement acceptables ("constituant actif B"),

lequel procédé comprend le mélange des constituants.

2. Procédé suivant la revendication 1, dans lequel le constituant actif A est le nédocromil sodique.

3. Procédé de préparation d'un sel de l'acide 9-éthyl-6,9-dihydro-4,6-dioxo-10-propyl-4H-pyrano[3,2-g]quinoléine-2,8-dicarboxylique avec un agent anticholinergique qui est basique ou qui est un cation ou est capable d'en former un,

lequel procédé comprend la réaction d'un sel approprié de l'acide 9-éthyl-6, 9-dihydro-4, 6-dioxo-10-propyl-4H-pyrano [3, 2 -g]quinoléine-2, 8-dicarboxylique avec un sel approprié de l'agent anticholinergique.

4. Procédé suivant la revendication 3, dans lequel l'agent cholinergique est basique,

lequel procédé comprend la réaction de l'acide 9-éthyl-6,9-dihydro-4,6-dioxo-10-propyl-4H-pyrano[3,2-g]quinoléine-2,8-dicarboxylique avec la base libre de l'agent cholinergique.

5. Procédé de préparation d'une composition pharmaceutique comprenant un mélange :

a) d'un ou plusieurs d'entre l'acide 9-éthyl-6,9-dihydro-4,6-dioxo-10-propyl-4H-pyrano[3,2-g]quinoléine-2,8-dicarboxylique et ses sels pharmaceutiquement acceptables ("constituant actif A") en combinaison avec

b) un ou plusieurs agents anticholinergiques ou leurs dérivés pharmaceutiquement acceptables ("constituant actif B"),

en mélange avec un excipient pharmaceutiquement acceptable,

lequel procédé comprend la mise en mélange des constituants actifs avec l'excipient.

6. Procédé de préparation d'une composition pharmaceutique comprenant un sel de l'acide 9-éthyl-6,9-dihydro-4,6-dioxo-10-propyl-4H-pyrano[3,2-g]quinoléine-2,8-dicarboxylique avec un agent anticholinergique qui est basique ou qui est un cation ou est capable d'en former un, en mélange avec un excipient pharmaceutiquement acceptable,

lequel procédé comprend la mise en mélange du sel avec l'excipient.

7. Procédé suivant la revendication 6, dans lequel la composition est une solution aqueuse.

8. Procédé suivant la revendication 5 ou 6, dans lequel la composition est une poudre mise ou non sous pression.